# EUROPÄISCHE PATENTSCHRIFT

(11) **EP 3 305 445 B1**
(45) Veröffentlichungstag und Bekanntmachung des Hinweises auf die Patenterteilung: **07.04.2021**
(21) Anmeldenummer: 17001609.1
(22) Anmeldetag: 28.09.2017
(51) Int. Cl.: B22F 3/105, G01N 27/407, G01N 30/88, B33Y 10/00, B33Y 30/00

(54) **VERFAHREN ZUR GENERATIVEN FERTIGUNG EINES 3-DIMENSIONALEN BAUTEILS**
METHOD FOR THE GENERATIVE PRODUCTION OF A THREE-DIMENSIONAL COMPONENT
PROCÉDÉ DE FABRICATION ADDITIVE D'UN COMPOSANT TRIDIMENSIONNEL

(30) Priorität: 10.10.2016 DE 102016012116
(43) Veröffentlichungstag der Anmeldung: 11.04.2018
(73) Patentinhaber: Linde GmbH, 82049 Pullach (DE)
(72) Erfinder: FORET, Pierre, 80796 München (DE)
(74) Vertreter: Gellner, Bernd

(56) Entgegenhaltungen:
- EP-A1- 2 774 703
- EP-A1- 3 075 470
- DE-U1-202016 004 832
- JP-A- H06 265 518
- US-A1- 2016 045 981
- US-A1- 2016 067 779

## Beschreibung

Die vorliegende Erfindung betrifft ein Verfahren zum generativen Fertigen eines dreidimensionalen Bauteils in einer Prozesskammer, wobei die Schritte
- Bereitstellen eines metallischen Ausgangsmaterials in der Prozesskammer
- Aufschmelzen des Ausgangsmaterials durch Energiezufuhr,
mehrfach wiederholt werden,
wobei in der Prozesskammer ein Prozessgas bereit gestellt wird, und wobei der Sauerstoffgehalt des Prozessgases bestimmt wird.

Mittels generativer Fertigungsverfahren ist es möglich, verschiedenste dreidimensionale Bauteile mit komplexer Geometrie herzustellen.

Beim 3D-Drucken werden beispielsweise dreidimensionale Werkstücke schichtweise aufgebaut. Der Aufbau erfolgt computergesteuert aus einem oder mehreren flüssigen oder festen Werkstoffen nach vorgegebenen Maßen und Formen (CAD). Beim Aufbau finden physikalische oder chemische Härtungs- oder Schmelzprozesse statt. Typische Werkstoffe für das 3D-Drucken sind Kunststoffe, Kunstharze, Keramiken und Metalle. 3D-Drucker werden in der Industrie und der Forschung eingesetzt. Daneben gibt es auch Anwendungen im Heim- und Unterhaltungsbereich sowie in der Kunst.

Der 3D-Druck ist ein generatives beziehungsweise additives Fertigungsverfahren. Die wichtigsten Techniken des 3D-Druckens sind das selektive Laserschmelzen und das Elektronenstrahlschmelzen für Metalle und das selektive Lasersintern für Polymere, Keramik und Metalle, die Stereolithografie und das Digital Light Processing für flüssige Kunstharze und das Polyjet-Modeling sowie das Fused Deposition Modeling für Kunststoffe und teilweise Kunstharze.

Ein weiteres generatives Verfahren ist das punktuelle Aufschmelzen und Erstarren. Bei dieser Art von Verfahren wird Metallpulver oder Metalldraht schichtweise aufgeschmolzen und erstarrt, sodass ein dreidimensionales Bauteil generiert werden kann. Durch die lokal begrenzte Energieeinbringung mittels Laserstrahl ist die Größe des ausgebildeten Schmelzbades gering. Somit besteht die Möglichkeit, filigrane Strukturen zu erzeugen. Entsprechende Verfahren werden als Laser Engineered Net Shaping (LENS), als Direct Metal Deposition (DMD), als Laser Additive Manufacturing (LAM), als Selective Laser Melting (SLM), als Laser Metal Fusion (LFM) oder als Laser Metal Deposition (LMD) kommerziell vertrieben.

Beim lokalen Laser-Sintern oder Schmelzen wird zwischen indirekten und direkten Verfahren unterschieden.

Selektives Laser-Sintern (SLS) ist ein Verfahren, um räumliche Strukturen durch Sintern aus einem pulverförmigen Ausgangsstoff herzustellen. Lasersintern ist ein generatives Schichtbauverfahren: das Werkstück wird Schicht für Schicht aufgebaut. Durch die Wirkung der Laserstrahlen können so beliebige dreidimensionale Geometrien auch mit Hinterschneidungen erzeugt werden, z.B. Werkstücke, die sich in konventioneller mechanischer oder gießtechnischer Fertigung nicht herstellen lassen.

Beim selektiven Laser-Sintern (SLS/LMF) wird auf einer Arbeitsfläche (Bauplattform) eine Schicht Pulverwerkstoff aufgetragen. Das lose Pulver wird durch einen Laserstrahl punktuell aufgeschmolzen. Dabei werden die Pulverpartikel je nach verwendetem Werkstoff in der Schicht und mit der darunter liegenden Schicht verbunden. Für die Herstellung metallischer Bauteile können zwei grundsätzliche Entwicklungsrichtungen differenziert werden. Neben der direkten Verfestigung metallischer Pulverwerkstoffe durch Laserstrahlung (direktes Metall-Laser-Sintern) hat sich bereits frühzeitig die Herstellung metallischer Bauteile über eine Kombination aus SLS von Kunstsoff ummanteltem Metallpulver mit nachträglicher thermischer Behandlung (IMLS) etabliert.

Beim direkten Metall-Laser-Sintern (DMLS) werden entweder ein- oder mehrkomponentige Metallwerkstoffe verwendet. Insbesondere werden dabei DMLS-Mehrkomponentenpulver verwendet, die aus verschiedenen Legierungselementen bestehen. Die im Pulver enthaltene niedrig schmelzende Komponente wird durch einen Laserstrahl aufgeschmolzen und umfließt die hochschmelzende Komponente, die als Strukturgeber dient.

Beim Electron Beam Melting (EBM) entspricht der Prozessablauf im Wesentlichen dem der Laser-basierten Verfahren. Loses Metallpulver, im Pulverbett oder über eine Düse, oder Draht wird dabei punktuell aufgeschmolzen und erstarrt anschließend in der gewünschten Kontur. Die dafür erforderliche Energie wird durch einen Elektronenstrahl eingebracht. Das Verfahren erfolgt meistens in einer Inertgas-gefluteten UnterdruccKammer.

Demgemäß werden bei generativen Fertigungsverfahren ein Pulverbett, eine Pulverzuführung oder eine Drahtzuführung verwendet, wobei diese Ausgangsmaterialien dann mittels Energiezufuhr, beispielsweise mittels Laserstrahl, Elektronenstrahl oder Plasma-/Lichtbogen, aufgeschmolzen und anschließend verfestigt werden. Weiterhin werden bei den generativen Fertigungsverfahren Inertoder Aktivgase als Prozessgase verwendet.

Die Qualität des hergestellten Bauteils hängt von vielen Parametern ab, insbesondere vom Prozessgas, dessen Zusammensetzung, Reinheit, Strömungsgeschwindigkeit, usw. Ein wesentlicher Faktor ist hierbei der Sauerstoffgehalt des Prozessgases, der Einfluss auf die Bauteilqualität besitzt.

Generative Fertigungsververfahren finden häufig in einer Prozesskammer statt, die mit einem Prozessgas gefüllt ist. Üblicherweise wird dabei ein Inertgas verwendet, bei dem die Verunreinigungen strikt kontrolliert werden müssen. Beispielsweise darf der Sauerstoffgehalt einen bestimmten Schwellenwert nicht übersteigen.

Im Stand der Technik wird beispielsweise vor Beginn des eigentlichen Fertigungsprozesses die Prozesskammer so lange mit Argon gespült, bis der Sauerstoffgehalt unter einem vorgegebenen Grenzwert von beispielsweise 0,1 % liegt. Erst dann wird mit der generativen Fertigung begonnen. Während des Fertigungsprozesses wird der Druck in der Prozesskammer kontrolliert und weiteres Argon zugeführt, wenn der Druck unter einen Mindestdruck fällt. Es wird ein leichter Argon-Überdruck in der Prozesskammer aufrechterhalten, um den Eintritt von Umgebungsluft zu verhindern. Auf diese Weise soll der Sauerstoffgehalt unter dem vorgegebenen Grenzwert von beispielsweise 0,1 % gehalten werden.

Bei diesem bekannten Verfahren wird der Sauerstoffgehalt nicht oder nicht exakt bestimmt und auch nicht kontrolliert. Das Verfahren erlaubt damit keine reproduzierbaren Prozessbedingungen.

Die DE 20 2016 004 832 U1 offenbart ein Gasüberwachungssystem zur Überwachung von Gaskonzentrationen in einer additiven Fertigungskammer. Das Gasüberwachungssystem umfasst einen ersten und einen zweiten Gassensor, wobei die beiden Gassensoren baugleich ausgeführt sind.

Die EP 2774703 A1 offenbart eine Vorrichtung zum additiven Fertigen, wobei zwei Sauerstoffsensoren vorgesehen sind. Einer der Sauerstoffsensoren dient zur Überwachung der Sauerstoffkonzentration im Prozessraum, der andere Sauerstoffsensor regelt die Bestrahlungsquelle und unterbricht die Bestrahlung, wenn der Sauerstoffgehalt zu hoch ist.

Aufgabe der vorliegenden Erfindung ist es daher ein generatives Fertigungsverfahren bereitzustellen, welches eine bessere und insbesondere reproduzierbare Einstellung und Kontrolle der Prozessgasatmosphäre in der Prozesskammer erlaubt.

Diese Aufgabe wird durch ein Verfahren gemäß Anspruch 1 gelöst.

Unter einem generativen Fertigen wird im Rahmen der vorliegenden Erfindung das lagenweise bzw. schichtweise Aufbauen eines dreidimensionalen Bauteils unter Verwendung eines Pulverbettes, einer Pulverzuführung oder einer Drahtzuführung, welche als Ausgangsmaterial dienen, und durch Energiezufuhr, beispielsweise mittels Laserstrahl, Elektronenstrahl oder Plasma bzw. Lichtbogen, aufgeschmolzen werden, verstanden. Diesbezüglich wird auf die in der Beschreibungseinleitung genannten generativen Fertigungsverfahren (3D-Drucken bzw. bei der Verfestigung durch chemische Aktivatoren, Aufschmelzen und Erstarren (Laser Engineered Net Shaping (LENS), als Direct Metal Deposition (DMD) oder als Laser Additive Manufacturing (LAM)), lokalen Sintern oder Schmelzen, (Laser-Sintern (SLS)) Metall-Laser-Sintern (DMLS), Metall-Laser-Sintern (IMLS), Electron Beam Melting (EBM)) Bezug genommen.

Im Rahmen von der Erfindung vorausgegangenen Versuchen wurde festgestellt, dass der Sauerstoffgehalt des Prozessgases einen entscheidenden Einfluss auf die Bauteilqualität hat. Je nachdem, welche Qualitätsanforderungen an das herzustellende Bauteil gestellt werden, ist daher eine unterschiedlich starke Kontrolle des Sauerstoffgehalts notwendig.

Der Sauerstoffgehalt des Prozessgases wird erfindungsgemäß mittels einer sogenannten Lambdasonde als erstem Sauerstoffsensor bestimmt. Eine Lambdasonde vergleicht den Sauerstoffgehalt im Prozessgas mit dem bekannten Sauerstoffgehalt eines Referenzgases. Dabei nutzt man die Eigenschaft von bestimmten Keramiken aus, Sauerstoffionen elektrolytisch transportieren zu können. Wird eine Seite der Keramik einem Referenzgas und die andere Seite der Keramik einem Prozessgas ausgesetzt, so entsteht eine Spannung, welche ein Maß für den unterschiedlichen Sauerstoffpartialdruck der beiden Gase ist. Bei bekanntem Referenzgas lässt sich daraus der Sauerstoffgehalt des Prozessgases ermitteln.

Die elektrolytische Leitfähigkeit vieler Keramiken tritt jedoch nur bei erhöhten Temperaturen von beispielsweise 700 °C auf. Die Lambdasonde und das Prozessgas werden daher für die Messung des Sauerstoffgehalts entsprechend aufgeheizt. Bei 700°C reagiert der Sauerstoff des Prozessgases mit etwaigem in dem Prozessgas befindlichen Wasserstoff zu Wasser. Das heißt, der Sauerstoffgehalt des Prozessgases wird durch dessen Messung gesenkt. Der von der Lambdasonde bestimmte Wert für den Sauerstoffgehalt ist daher niedriger als der tatsächliche Sauerstoffgehalt des Prozessgases.

Für Anwendungen mit geringen Anforderungen an die Qualität des Bauteils und die Reproduzierbarkeit des Prozesses ist die Verwendung einer Lambdasonde aber durchaus ausreichend. Sollen dagegen qualitativ hochwertige Bauteile, beispielsweise sicherheitsrelevante Bauteile in der Luftfahrtindustrie, hergestellt werden, ist die Reproduzierbarkeit des Verfahrens unabdingbar. In diesem Fall ist eine exakte Bestimmung und Kontrolle des Sauerstoffgehalts des Prozessgases nötig.

Zur genaueren Bestimmung des Sauerstoffgehalts des Prozessgases wird daher ein zweiter Sauerstoffsensor eingesetzt. Erfindungsgemäß werden je nach den Qualitätsanforderungen an das Bauteil entweder der erste Sauerstoffsensor oder der zweite Sauerstoffsensor oder beide Sauerstoffsensoren verwendet, um den Sauerstoffgehalt des Prozessgases zu bestimmen.

Als erster Sauerstoffsensor wird erfindungsgemäß eine Lambdasonde eingesetzt. Eine Lambdasonde ist kostengünstig und besitzt eine kurze Ansprechzeit. Das heißt, die Lambdasonde reagiert schnell auf Änderungen des Sauerstoffgehalts. Wie oben bereits ausgeführt, hat eine Lambdasonde allerdings den Nachteil, dass der gemessene Sauerstoffgehalt nicht mit dem tatsächlichen Sauerstoffgehalt übereinstimmt. Dies liegt darin begründet, dass die Lambdasonde während des Messvorgangs, d.h. während der Bestimmung des Sauerstoffgehalts, eine Reaktion des vorhandenen Sauerstoffs mit anderen Bestandteilen des Prozessgases hervorruft. Für Bauteile mit geringen Qualitätsanforderungen ist dies aber tolerierbar.

Als zweiter Sauerstoffsensor wird eine Mess- oder Aufnahmeeinrichtung verwendet, die den Sauerstoffgehalt des Gases mittels eines Verfahrens bestimmt, bei dem die übrigen Bestandteile des Prozessgases keinen Einfluss auf den ermittelten Wert für den Sauerstoffgehalt haben.

In einer bevorzugten Ausführungsform der Erfindung wird eine chemische Zelle, beispielsweise ein galvanischer oder amperometrischer Sensor, eingesetzt. Vorzugsweise wird ein Sensor als zweiter Sauerstoffsensor verwendet, der bei einer Temperatur von weniger als 500°C, weniger als 300 °C, weniger als 100°C oder weniger als 40°C arbeitet. In diesen Temperaturbereichen ist die Geschwindigkeit etwaiger Reaktionen von Sauerstoff mit anderen Bestandteilen des Prozessgases so gering, dass während der Messung praktisch keine Umsetzung von Sauerstoff stattfindet.

Der erste und der zweite Sauerstoffsensor können direkt in der Prozesskammer oder außerhalb der Prozesskammer vorgesehen sein. Entsprechend kann das Prozessgas, dessen Sauerstoffgehalt bestimmt wird, entweder in der Prozesskammer verbleiben oder ein Teil des Prozessgases wird aus der Prozesskammer abgezogen und dem ersten und/oder dem zweiten Sauerstoffsensor zugeführt, um dessen Sauerstoffgehalt zu ermitteln. Es ist weiterhin möglich, den ersten Sauerstoffsensor in der Prozesskammer und den zweiten Sauerstoffsensor außerhalb der Prozesskammer vorzusehen. Selbstverständlich ist dies auch umgekehrt möglich, d.h. der zweite Sauerstoffsensor befindet sich in der Prozesskammer und der erste Sauerstoffsensor befindet sich außerhalb der Prozesskammer.

In einer bevorzugten Ausführung der Erfindung wird ein Teil des Prozessgases aus der Prozesskammer abgezogen und wieder in die Prozesskammer zurückgeführt. Ein Teil des Prozessgases wird also im Kreislauf geführt. In diesem Fall kann die Bestimmung des Sauerstoffgehalts an dem der Prozesskammer entnommenen Teil des Prozessgases erfolgen. Je nach Ergebnis der Untersuchung des Sauerstoffgehalts wird der aus der Prozesskammer abgezogene Teil des Prozessgases wieder komplett oder teilweise in die Prozesskammer zurückgeführt oder anderweitig ausgeleitet.

Entscheidend für die Reproduzierbarkeit des generativen Fertigungsverfahrens und die Qualität des hergestellten Bauteils ist der Sauerstoffgehalt an der momentanen Bearbeitungsstelle, an der das Pulver aufgeschmolzen wird. Von Vorteil wird daher das Prozessgas in unmittelbarer Nähe zur Bearbeitungsstelle abgezogen und dem ersten und/oder zweiten Sauerstoffsensor zugeführt. Hierzu kann beispielsweise auf Höhe der Bearbeitungsebene, auf der sich das zuletzt aufgetragene Pulver befindet, ein Abzug für Prozessgas vorgesehen sein.

Ist eine Kreislaufströmung für das Prozessgas, entweder aus der Prozesskammer hinaus und wieder zurück in die Prozesskammer oder auch eine Kreislaufführung innerhalb der Prozesskammer vorgesehen, so ist es günstig, den für die Sauerstoffbestimmung vorgesehenen Teil des Prozessgases aus diesem Kreislaufstrom abzuziehen. Auf diese Weise wird sichergestellt, dass auch der Sauerstoffgehalt des Teils des Prozessgases bestimmt wird, der an der Bearbeitungsstelle vorbeiströmt.

Zusätzlich können auch noch weitere Parameter des in der Prozesskammer befindlichen Prozessgases oder des aus der Prozesskammer abgezogenen Stroms an Prozessgas bestimmt und gegebenenfalls mit einem Sollwert verglichen werden. Zum Beispiel können der Wasserdampfgehalt oder der Taupunkt des aus der Prozesskammer abgezogenen Prozessgases oder der Wasserdampfgehalt oder der Taupunkt der Gasatmosphäre in der Prozesskammer ermittelt werden und mit einem vorgegebenen Sollwert verglichen werden. In Abhängigkeit von dem Ergebnis des Vergleichs des ermittelten Wertes mit dem Sollwert wird zuvor aus der Prozesskammer abgezogenes Prozessgas entweder vollständig, teilweise oder überhaupt nicht in die Prozesskammer zurückgeführt. Je nach Ergebnis kann auch ein Teil des abgezogenen Stroms durch Inertgas, welches in die Prozesskammer eingeleitet wird, ersetzt werden.

Der bei einer Kreislaufführung zurückgeführte Gasstrom oder neu hinzugegebenes Prozessgas werden der Prozesskammer über einen oder mehrere Einlässe zugeführt, wobei die Einlässe vorzugsweise in einem unteren Bereich der Prozesskammer angeordnet sind. Auf diese Weise bleiben die Prozessparameter stabil und es sind homogene metallurgische Effekte während der Herstellung erzielbar. Insbesondere kann vorgesehen sein, dass zumindest ein Teil des Prozessgases durch das Ausgangsmaterial, das als Pulverbett vorliegt, hindurch zugeführt wird. Durch die unmittelbare Nähe des Bereichs, in dem das Prozessgas in die Prozesskammer eintritt, zum Pulverbett und zur Bearbeitungsstelle wird eine konstante Atmosphäre beim generativen Fertigen des Bauteils an der obersten Schicht des Bauteils erzeugt.

Das erfindungsgemäße Verfahren kann vorzugsweise ein Laser-Schmelz-Verfahren betreffen. Das Ausgangsmaterial wird dann mittels eines Laserstrahls lokal aufgeschmolzen.

Als Prozessgas ist vorzugsweise ein Inertgas, das bei gleicher Temperatur eine höhere Dichte als Luft aufweist, wie z.B. Argon, vorgesehen. Das Inertgas kann vorzugsweise eine Temperatur aufweisen, die niedriger als die Temperatur der in der Prozesskammer zu Beginn vorliegenden Luft ist. In beiden Fällen sammelt sich das Prozessgas im unteren Bereich der Prozesskammer, in dem sich auch die Bearbeitungsstelle befindet. Das schwerere gasförmige Argon drückt beispielsweise die leichtere Luft in den oberen Bereich der Prozesskammer, in dem zum Beispiel ein Auslass zum Abführen der Luft vorgesehen ist.

Es ist auch möglich, dem Prozessgas reaktive Komponenten, insbesondere reaktive Gase wie zum Beispiel CO oder CO2, oder gut wärmeleitende Gase, wie zum Beispiel He, zuzugeben.

Das Prozessgas kann in der Prozesskammer mittels zumindest einer Ventilatoreinrichtung verwirbelt werden. Auf diese Weise wird eine homogene Gaszusammensetzung über das gesamte Volumen der Prozesskammer bereitgestellt. Die entnommene Probe stellt somit eine gute Repräsentation der Zusammensetzung des Prozessgases in der Prozesskammer dar.

Auf die erfindungsgemäße Weise wird der Sauerstoffgehalt des Prozessgases in Abhängigkeit von der geforderten Qualität des Bauteils bestimmt. In einer Ausführungsform der Erfindung wird dem Prozessgas und/oder der Prozesskammer ein sauerstofffreies Gas zugeführt, wenn der gemessene Sauerstoffgehalt größer als ein vorgegebener Vergleichswert ist. Auf diese Weise kann der Sauerstoffgehalt des Prozessgases in der Prozesskammer unterhalb eines vorgegebenen Maximalwertes gehalten werden.

Die Erfindung ermöglicht zum einen die schnelle Herstellung von Bauteilen, an deren Qualität geringere Anforderungen gestellt werden und/oder bei denen es auf exakte Reproduzierbarkeit nicht ankommt. In diesen Fällen wird der Sauerstoffgehalt des Prozessgases mittels des ersten Sauerstoffsensors bestimmt. Der erste Sauerstoffsensor ist in der Regel einfacher aufgebaut und kostengünstiger als der zweite Sauerstoffsensor. Insbesondere ist es vorteilhaft, wenn der erste Sauerstoffsensor eine kürzere Ansprechzeit als der zweite Sauerstoffsensor besitzt.

Andererseits erlaubt die Erfindung auch die reproduzierbare Herstellung hochqualitativer Bauteile. In diesem Fall wird der Sauerstoffgehalt des Prozessgases mittels des zweiten Sauerstoffsensors bestimmt. Die Messung des zweiten Sauerstoffsensors ist unabhängig von den sonstigen Komponenten des Prozessgases. Es wird exakt der Sauerstoffgehalt gemessen, der auch vor Beginn der Messung im Prozessgas vorlag. Die Messung hat keinen Einfluss auf den Sauerstoffgehalt. Hierdurch wird eine exakte und reproduzierbare Einstellung des Sauerstoffgehalts ermöglicht. Die Eigenschaften auf diese Weise hergestellter Bauteile können exakt reproduziert werden.

Das erfindungsgemäße Verfahren hat sich beispielsweise bei der herstellung von Bauteilen aus AISiMg, zum Beispiel AISi7Mg oder AISi10Mg, bewährt. Auch bei der Verarbeitung von Titanpulver oder titanhaltigen Pulvern kann der negative Einfluss von Sauerstoff auf die Qualität der gefertigten Bauteile ausgeschaltet werden.

Die Erfindung sowie weitere Einzelheiten der Erfindung werden im Folgenden anhand von in den Zeichnungen schematisch dargestellten Ausführungsbeispielen näher erläutert. Hierbei zeigen:
- Figur 1: eine erste erfindungsgemäße Vorrichtung zum generativen Fertigen eines 3-dimensionalen Bauteils und
- Figur 2: eine alternative Ausführungsform der Erfindung.

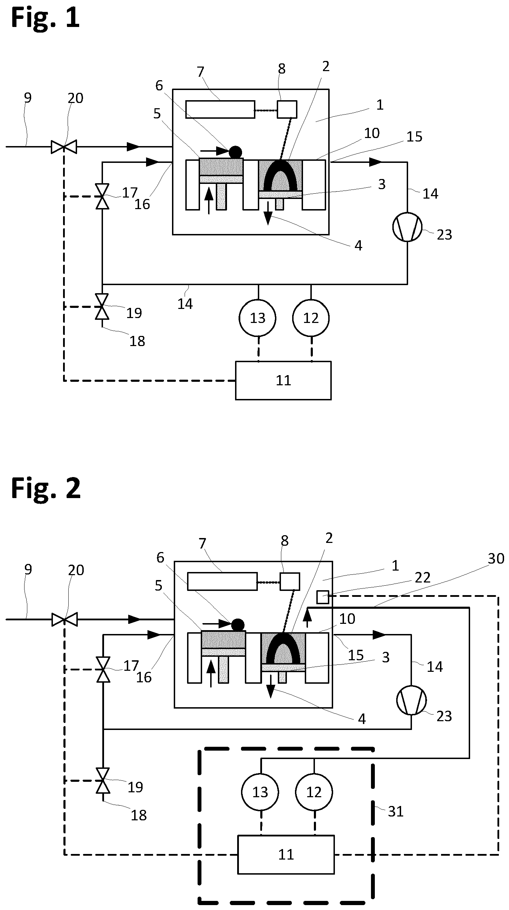

Figur 1 zeigt eine schematische Darstellung einer Vorrichtung zur Durchführung des erfindungsgemäßen Verfahrens.

Im Folgenden wird eine Vorrichtung zum generativen Fertigen eines dreidimensionalen Bauteils beschrieben. Wie bereits vorstehend erwähnt, ist das erfindungsgemäße Verfahren aber nicht auf die dargestellte Vorrichtung zum generativen Fertigen von dreidimensionalen Bauteilen beschränkt.

Die Vorrichtung ist eine Laser-Schmelz-Vorrichtung. Die Laser-Schmelz-Vorrichtung umfasst eine Prozesskammer 1, die als Bauraum für das dreidimensionale Bauteil 2 dient.

In der Prozesskammer 1 befindet sich eine Bauplattform 3 zur Aufnahme des zu fertigenden Bauteils 2 angeordnet. Die Bauplattform 3 weist eine Höhenverstelleinrichtung 4 auf, mittels der die Bauplattform 3 in vertikaler Richtung einstellbar ist.

Weiterhin umfasst die Vorrichtung 1 einen Vorratsbehälter 5. Der Vorratsbehälter 5 ist zur Aufnahme eines verfestigbaren pulverförmigen Ausgangsmaterials ausgebildet.

Weiterhin ist eine Auftrageinrichtung 6 zum Aufbringen des Ausgangsmaterials auf die Bauplattform 3 vorgesehen. Eine solche Auftrageinrichtung 6 ist in horizontaler Richtung parallel zur Arbeitsebene 10 beweglich.

Zudem ist ein Laser 7 zum Erzeugen eines Laserstrahls vorgesehen. Ein von dem Laser 7 erzeugter Laserstrahl wird über eine Umlenkeinrichtung 8 umgelenkt und durch eine Fokussiereinrichtung (nicht dargestellt) auf einen vorbestimmten Punkt unmittelbar unterhalb oder in der Arbeitsebene 10 fokussiert. Mittels der Umlenkeinrichtung 8 kann der Verlauf des Laserstrahls derart verändert werden, dass er die dem Querschnitt des herzustellenden Objekts 2 entsprechenden Stellen der aufgetragenen Schicht aufschmilzt.

Zudem ist eine Prozessgaszuführeinrichtung 9 vorgesehen, mittels der die Prozesskammer 1 mit einem Prozessgas beaufschlagbar ist.

Die Prozessgaszuführeinrichtung 9 weist einen oder mehrere Vorratsbehälter für das Prozessgas oder einzelne Bestandteile des Prozessgases auf, wobei der Prozessgasvorratsbehälter (nicht dargestellt) über einen bzw. mehrere Leitungsabschnitte mit in die Prozesskammer mündenden Einlässen (nicht dargestellt) verbunden ist. Die Einlässe, z.B. eine oder mehrere Düsen, zum Zuführen von Prozessgas sind in einem unteren Bereich der Prozesskammer 1 angeordnet. Die zugeführte Gasmenge kann mittels eines Regelventils 20 eingestellt werden. Vorzugsweise ist zumindest eine Düse der Prozessgaszuführeinrichtung im Bereich des Bodens der Prozesskammer 1 oder auf einem Fünftel, einem Viertel, der Hälfte, zwei Drittel oder drei Viertel der Höhe zwischen dem Boden der Prozesskammer 1 und der Arbeitsebene 10 oder in etwa auf Höhe der Arbeitsebene 10 angeordnet.

Als Prozessgas ist vorzugsweise ein Inertgas, wie z.B. Argon, vorgesehen, das bei gleicher Temperatur eine höhere Dichte als Luft aufweist.

Ebenfalls in einem unteren Bereich der Prozesskammer ist eine Ventilatoreinrichtung (nicht dargestellt) angeordnet. Es können auch mehrere Ventilatoreinrichtungen vorgesehen sein.

Weiter ist eine Kreislaufführung 14 eines Teils des Prozessgases vorgesehen. Ein Teil des Prozessgases kann über einen Auslass 15 aus der Prozesskammer 1 abgezogen, durch die Kreislaufleitung 14 geführt und über den Einlass 16 wieder in die Prozesskammer 1 zurückgeführt werden. Die Kreislaufführung des Prozessgases erfolgt beispielsweise mittels eines Gebläses oder eines Verdichters 23. In der Kreislaufleitung 14 ist ferner ein Regelventil 17 vorgesehen, mittels dessen die in die Prozesskammer 1 zurückgeführte Gasmenge geregelt werden kann. Außerdem ist eine Leitung 18 vorgesehen, welche von der Kreislaufleitung 14 abzweigt und es erlaubt, das durch die Kreislaufleitung 14 geführte Prozessgas abzuziehen. Die Leitung 18 ist ebenfalls mit einem Regelventil 19 versehen.

Weiterhin umfasst die Vorrichtung eine Steuereinrichtung 11 zum Steuern des Regelventils 20 der Prozessgaszuführeinrichtung 9 und der Regelventile 17 und 19. Die Steuereinrichtung 11 kann eine oder vorzugsweise zwei Regeleinrichtungen (nicht dargestellt) mit einem geschlossenen Regelkreis umfassen. Die Regeleinrichtungen können auch einen P-Regler, einen I-Regler, einen D-Regler und Kombinationen daraus, wie z.B. einen PID-Regler umfassen.

Außerdem sind ein zweiter Sauerstoffsensor 12 zur Bestimmung des Sauerstoffgehalts des durch die Kreislaufleitung 14 geführten Prozessgases und eine Lambdasonde 13 zur Bestimmung des Sauerstoffgehalts des durch die Kreislaufleitung 14 geführten Prozessgases vorgesehen. Der zweite Sauerstoffsensor 12 und die Lambdasonde 13 sind an die Steuereinrichtung 11 angeschlossen.

Im Folgenden wird ein erfindungsgemäßes Verfahren anhand eines Ausführungsbeispiels beschrieben.

Argon wird als Prozessgas in einem unteren Bereich der Prozesskammer 1 zugeführt. Dadurch, dass die Prozessgaszuführeinrichtung 9 das Prozessgas auf Höhe der Arbeitsebene 10 oder darunter zugeführt wird, füllt sich die Prozesskammer 1 von unten nach oben mit dem Prozessgas.

Auf diese Weise drückt das schwerere gasförmige Argon die leichtere Luft in den oberen Bereich der Prozesskammer 1, in dem ein (nicht dargestellter) Auslass zum Abführen der Luft vorgesehen ist.

Das in der Prozesskammer 1 befindliche Prozessgas kann gegebenenfalls mittels einer Ventilatoreinrichtung in der Prozesskammer 1 verwirbelt werden. Durch die Verwirbelung werden Verunreinigungen aus den Toträumen der Prozesskammer entfernt. Zudem wird eine homogene Gaszusammensetzung über das gesamte Volumen der Prozesskammer bereitgestellt. Zusätzlich kann der Prozesskammer 1 sauberes Prozessgas über die Prozessgaszuführeinrichtung 9 zugeführt werden.

Ein metallisches Ausgangsmaterial wird auf der Bauplattform 3 in Form eines Pulverbetts mittels der Auftrageinrichtung 6 aufgebracht bzw. bereitgestellt. Alternativ kann das metallische Ausgangsmaterial auch mittels einer Pulverzuführung oder einer Drahtzuführung zugeführt werden.

Anschließend wird das Ausgangsmaterial mittels des Lasers 7 aufgeschmolzen. Die beiden Schritte "Bereitstellen von Ausgangsmaterial auf der Bauplattform 3" und "Aufschmelzen des Ausgangsmaterials" werden mehrfach wiederholt, wodurch das Bauteil schichtweise aufgebaut wird.

Der Sauerstoffgehalt des Prozessgases sollte aber während des Fertigungsprozesses unterhalb eines vorgegebenen Maximalwertes bleiben, um unerwünschten Oxidationen vorzubeugen. Erfindungsgemäß wird daher der Sauerstoffgehalt des Prozessgases überwacht. Hierzu wird eine Probe des durch die Kreislaufleitung 14 geführten Prozessgases der Lambdasonde 13 zugeführt und mittels der Lambdasonde 13 wird der Sauerstoffgehalt der Probe bestimmt. Der so ermittelte Wert für den Sauerstoffgehalt wird der Steuereinrichtung 11 übermittelt.

In der Lambdasonde 13 wird die Probe erwärmt, wobei Wasserstoff und Sauerstoff zu Wasser rekombinieren können. Der von der Lambdasonde 13 ermittelte Wert für den Sauerstoffgehalt der Probe stimmt daher nicht exakt mit dem tatsächlichen Sauerstoffgehalt des Prozessgases überein. Für viele Anwendungen, bei denen es nicht auf exakte Reprozierbarkeit ankommt, ist diese Ungenauigkeit jedoch akzeptabel.

Sollen jedoch Bauteile mit reproduzierbaren Eigenschaften hergestellt werden, beispielsweise sicherheitsrelevante Bauteile oder Bauteile, die spezielle mechanische Mindestanforderungen erfüllen müssen, sind auch geringe Schwankungen des Sauerstoffgehalts nicht hinnehmbar.

Aus diesem Grund wird zusätzlich oder alternativ zu der Messung des ersten Sauerstoffsensors der Sauerstoffgehalt des Prozessgases bzw. des im Kreislauf geführten Prozessgases auch mittels des zweiten Sauerstoffsensors bestimmt. Hierzu wird dem Prozessgas eine zweite Probe entnommen und mittels des zweiten Sauerstoffsensors 12 wird deren Sauerstoffgehalt bestimmt. Als zweiter Sauerstoffsensor 12 wird eine Messvorrichtung oder ein Messelement verwendet, welches ohne Wechselwirkung mit den übrigen Bestandteilen des Prozessgases arbeitet. Insbesondere wird der gemessene Wert für den Sauerstoffgehalt durch die Messung selbst nicht verfälscht.

Der vom Messsensor 12 ermittelte Wert für den Wasserstoffgehalt wird ebenfalls der Steuereinrichtung 11 zugeführt. In Abhängigkeit von dem ermittelten Wert für den Sauerstoffgehalt wird dann die Prozessgaszusammensetzung in der Prozesskammer 1 geregelt. Hierzu kann ein Teil der ursprünglichen Prozessgasatmosphäre über Leitung 18 abgeführt und / oder die Zusammensetzung und/oder Menge des über die Prozessgaszuführeinrichtung 9 zugeführten Prozessgases geändert werden.

In Figur 2 ist eine andere Ausführungsform der Erfindung dargestellt. Gleiche Bauteile sind in beiden Figuren mit denselben Bezugszeichen versehen.

Die Ausführung nach Figur 2 unterscheidet sich im Wesentlichen von der nach Figur 1 darin, dass ein separater Abzug 30 zum Abziehen von Prozessgas aus der Prozesskammer 1 und in eine Analyseeinrichtung 31 vorgesehen ist. Der Abzug 30 befindet sich in unmittelbarer Nähe der Bearbeitungsstelle, an der das Pulver mittels des Lasers 7 aufgeschmolzen wird, und innerhalb der Kreislaufströmung, die das Prozessgas durch die Prozesskammer 1 und die Kreislaufleitung 14 führt. Über den Abzug 30 wird ein Teil des Prozessgases dem ersten und/oder dem zweiten Sauerstoffsensor 12, 13 zugeführt.

Die Ausführung nach Figur 2 erlaubt auch die einfache Nachrüstung bestehender Anlagen zum generativen Fertigen. Es muss lediglich die Abzugsleitung 30 in das Innere der Prozesskammer 1 geführt werden. Weitere direkte Eingriffe in die Anlage sind nicht erforderlich. Eine externe Analyseeinrichtung 31 mit dem dem ersten und dem zweiten Sauerstoffsensor 12, 13 wird an den Abzug 30 angeschlossen und mit der Steuereinrichtung 11 der bestehenden Anlage oder mit einer externen Steuereinrichtung verbunden. Je nach den geforderten Qualitätsansprüchen schaltet die Analyseeinrichtung 31 auf eine Sauerstoffmessung mittels des ersten Sauerstoffsensors 12 oder mittels des zweiten Sauerstoffsensors 13 um.

### Bezugszeichenliste:

- 1: Prozesskammer
- 2: Bauteil
- 3: Bauplattform
- 4: Höhenverstelleinrichtung
- 5: Vorratsbehälter
- 6: Auftrageinrichtung
- 7: Laser
- 8: Umlenkeinrichtung
- 9: Prozessgaszuführeinrichtung
- 10: Arbeitsebene
- 11: Steuereinrichtung
- 12: Messsensor
- 13: Lambdasonde
- 14: Kreislaufleitung
- 15: Auslass
- 16: Einlass
- 17: Regelventil
- 18: Leitung
- 19: Regelventil
- 20: Regelventil
- 21: Wasserdampfmessung
- 22: Sensor
- 23: Gebläse
- 30: Abzug
- 31: Analyseeinrichtung

## Patentansprüche

1. Verfahren zum generativen Fertigen eines dreidimensionalen Bauteils in einer Prozesskammer, wobei die Schritte
- Bereitstellen eines metallischen Ausgangsmaterials in der Prozesskammer
- Aufschmelzen des Ausgangsmaterials durch Energiezufuhr,
mehrfach wiederholt werden,
wobei in der Prozesskammer ein Prozessgas bereit gestellt wird, und wobei der Sauerstoffgehalt des Prozessgases bestimmt wird,
**dadurch gekennzeichnet,**
**dass** ein erster Sauerstoffsensor und ein zweiter Sauerstoffsensor vorgesehen sind, wobei der erste Sauerstoffsensor eine Lambdasonde ist und wobei als zweiter Sauerstoffsensor eine Mess- oder Aufnahmeeinrichtung verwendet wird, die den Sauerstoffgehalt des Prozessgases mittels eines Verfahrens bestimmt, bei dem die übrigen Bestandteile des Prozessgases keinen Einfluss auf den ermittelten Wert für den Sauerstoffgehalt haben, und dass in Abhängigkeit von der gewünschten Qualität des herzustellenden Bauteils der Sauerstoffgehalt des Prozessgases mittels des ersten Sauerstoffsensors und/oder mittels des zweiten Sauerstoffsensors bestimmt wird.

2. Verfahren nach einem der vorhergehenden Ansprüche, **dadurch gekennzeichnet, dass** der zweite Sauerstoffsensor eine chemische Zelle ist.

3. Verfahren nach einem der vorhergehenden Ansprüche, **dadurch gekennzeichnet, dass** der erste und/oder der zweite Sauerstoffsensor in der Prozesskammer angeordnet sind.

4. Verfahren nach einem der vorhergehenden Ansprüche, **dadurch gekennzeichnet, dass** ein Teil des Prozessgases aus der Prozesskammer abgezogen und der Sauerstoffgehalt des aus der Prozesskammer abgezogenen Teils des Prozessgases bestimmt wird.

5. Verfahren nach Anspruch 4, **dadurch gekennzeichnet, dass** der abgezogene Teil des Prozessgases wieder in die Prozesskammer zurückgeführt wird.

6. Verfahren nach einem der vorhergehenden Ansprüche, **dadurch gekennzeichnet, dass** dem Prozessgas ein sauerstofffreies Gas zugeführt wird, wenn der mit dem ersten und/oder zweiten Sauerstoffsensor bestimmte Wert für den Sauerstoffgehalt größer als ein vorgegebener Vergleichswert ist.

7. Verfahren nach einem der vorhergehenden Ansprüche, **dadurch gekennzeichnet, dass** die Energie zum Aufschmelzen des Ausgangsmaterials mit einem Laser zugeführt wird.

## Claims

1. Method for the generative production of a three-dimensional component in a process chamber, wherein the steps of
- providing a metallic starting material in the process chamber
- melting the starting material by supplying energy,
are repeated several times,
wherein a process gas is provided in the process chamber and wherein the oxygen content of the process gas is determined,
**characterized in that**
a first oxygen sensor and a second oxygen sensor are provided, wherein the first oxygen sensor is a lambda probe, and wherein a measuring or recording device is used as second oxygen sensor which determines the oxygen content of the process gas by means of a method in which the remaining constituents of the process gas have no influence on the determined value for the oxygen content, and **that** the oxygen content of the process gas is determined by means of the first oxygen sensor and/or by means of the second oxygen sensor as a function of the desired quality of the component to be produced.

2. Method according to any one of the preceding claims, **characterized in that** the second oxygen sensor is a chemical cell.

3. Method according to any one of the preceding claims, **characterized in that** the first and/or the second oxygen sensor are arranged in the process chamber.

4. Method according to any one of the preceding claims, **characterized in that** a portion of the process gas is extracted from the process chamber and the oxygen content of the portion of the process gas extracted from the process chamber is determined.

5. Method according to claim 4, **characterized in that** the extracted portion of the process gas is returned to the process chamber.

6. Method according to any one of the preceding claims, **characterized in that** an oxygen-free gas is supplied to the process gas if the value for the oxygen content determined with the first and/or the second oxygen sensor is greater than a specified comparison value.

7. Method according to any of the preceding claims, **characterized in that** the energy for melting the starting material is supplied by a laser.

## Revendications

1. Procédé de fabrication additive d'un composant tridimensionnel dans une chambre de traitement, dans lequel les étapes de
- fourniture d'un matériau de base métallique dans la chambre de traitement
- fusion du matériau de base par apport d'énergie,
sont répétées plusieurs fois,
dans lequel un gaz de traitement est fourni dans la chambre de traitement et dans lequel la teneur en oxygène du gaz de traitement est déterminée,
**caractérisé en ce**
**qu**'un premier capteur d'oxygène et un deuxième capteur d'oxygène sont prévus, le premier capteur d'oxygène étant une sonde lambda et un dispositif de mesure ou de réception étant utilisé comme deuxième capteur d'oxygène, lequel détermine la teneur en oxygène d'un gaz de traitement au moyen d'un procédé dans lequel les autres composants du gaz de traitement n'ont aucune influence sur la valeur déterminée pour la teneur en oxygène et **que,** en fonction de la qualité souhaitée du composant à fabriquer, la teneur en oxygène du gaz de traitement est déterminée au moyen du premier capteur d'oxygène et/ou au moyen du deuxième capteur d'oxygène.

2. Procédé selon l'une quelconque des revendications précédentes, **caractérisé en ce que** le deuxième capteur d'oxygène est une cellule chimique.

3. Procédé selon l'une quelconque des revendications précédentes, **caractérisé en ce que** le premier et/ou le deuxième capteur d'oxygène sont disposés dans la chambre de traitement.

4. Procédé selon l'une quelconque des revendications précédentes, **caractérisé en ce qu'une** partie du gaz de traitement est retirée de la chambre de traitement et la teneur en oxygène de la partie du gaz de traitement retirée de la chambre de traitement est déterminée.

5. Procédé selon la revendication 4, **caractérisé en ce que** la partie du gaz de traitement retirée est ramenée dans la chambre de traitement.

6. Procédé selon l'une quelconque des revendications précédentes, **caractérisé en ce qu'un** gaz sans oxygène est apporté au gaz de traitement, lorsque la valeur pour la teneur en oxygène déterminée au moyen du premier et/ou du deuxième capteur d'oxygène est supérieure à une valeur de comparaison prédéfinie.

7. Procédé selon l'une quelconque des revendications précédentes, **caractérisé en ce que** l'énergie pour la fusion du matériau de base est apportée au moyen d'un laser.
